# EUROPEAN PATENT APPLICATION

(11) **EP 1 985 234 A1**
(43) Date of publication of application: **29.10.2008**
(21) Application number: 07713736.2
(22) Date of filing: 31.01.2007
(51) Int. Cl.: A61B 5/151, A61B 5/157

(54) **LANCET**

(30) Priority: 01.02.2006 JP 2006024951
(71) Applicant: Arkray, Inc., Kyoto-shi, Kyoto 601-8045 (JP)
(72) Inventor: DOI, Shigeru, Kyoto-shi, Kyoto 6018045 (JP)
(74) Representative: HOFFMANN EITLE
(86) International application number: PCT/JP2007/051565
(87) International publication number: WO 2007/088875

(57) **Abstract**

The present invention relates to a lancet 1 including a puncture body 4 with a puncture needle 41, and a hollow member 3 having an internal space 30 for accommodating the puncture body 4. The lancet 1 further includes a sealing part 5 for sealing and accommodating the puncture needle 41 arranged in the hollow member 3. The hollow member 3 has an opening 32 for allowing the movement of the puncture needle 41 when the puncture needle 41 is moved in a puncturing direction N1. The sealing part 5 includes a seal member securely attached so as to cover the opening 32.

## Description

### TECHNICAL FIELD

The present invention relates to a lancet used by being attached to a puncture device when puncturing the skin to draw blood.

### BACKGROUND ART

A method using a biosensor is conventionally known as a method of measuring the blood sugar. One example is a method of automatically measuring the blood sugar in a blood sugar measuring device by having the user attach a biosensor to a portable blood sugar measuring device that can be carried around, and spot the blood drawn from the skin to the biosensor (see e.g., Patent Document 1).

A puncture device 9A and a lancet 9B shown in Fig. 19 and Fig. 20 are used to draw blood from the skin (e.g., Patent Document 2). The puncture device 9A includes a lancet holder 90A for holding the lancet 9B. The lancet holder 90A is movable in a N1 direction towards the skin Sk by an elastic force of a coil spring 91A, and is configured to stick a puncture needle 90B of the lancet 9B into the skin Sk by being moved in the N1 direction towards the skin Sk while holding the lancet 9B.

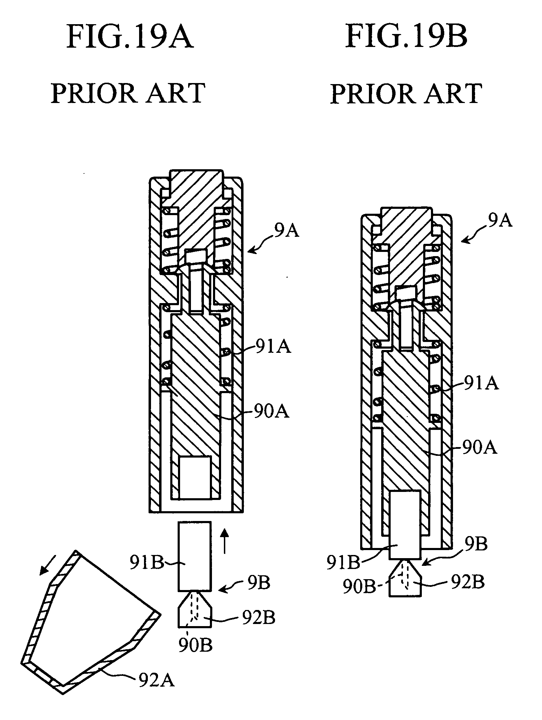

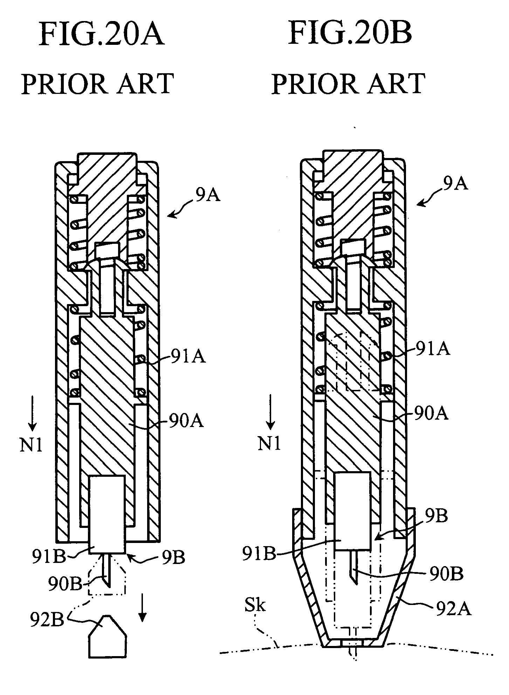

The lancet 9B includes a main body 91B insert molded with the puncture needle 90B, and a cap 92B for covering a distal end of the puncture needle 90B. When using such lancet 9B, the lancet 9B is attached to the lancet holder 90A with a cover 92A of the puncture device 9A removed, as shown in Fig. 19A and Fig. 19B. Then, the cover 92A is attached after detaching the cap 92B of the lancet 9B, and the lancet 9B is moved in the N1 direction along with the lancet holder 90A to puncture the skin Sk, as shown in Fig. 20A and Fig. 20B.

However, the cap 92B needs to be detached after being attached to the lancet holder 90A in the lancet 9B as shown in Fig. 20A in order to carry out the puncturing operation. Thus, the user is forced to perform a troublesome task. Furthermore, the cover 92A of the puncture device 9A needs to be detached as shown in Fig. 19A and Fig. 20B when attaching the lancet 9B to the lancet holder 90A and detaching the lancet 9B from the lancet holder 90A. This also forces the user to perform a troublesome task.

Moreover, the distal end of the puncture needle 90B is exposed when the cap 92B is detached from the lancet 9B, as clearly illustrated in Fig. 20A. Thus, when detaching the cap 92B from the lancet 9B and attaching the cover 92A to the puncture device 9A or when detaching the used lancet 9B after puncturing, the user may feel a sense of fear.

Patent Document 1: Japanese Unexamined Patent Publication No. 2003-156469
Patent Document 2: Japanese Unexamined Patent Publication No. 05-285127

### DISCLOSURE OF THE INVENTION

### PROBLEMS TO BE SOLVED BY THE INVENTION

The present invention aims to provide a lancet which can be used with a simple operation without the user feeling a sense of fear.

### MEANS FOR SOLVING THE PROBLEMS

The present invention provides a lancet including a puncture body with a puncture needle, and a hollow member having an internal space for accommodating the puncture body, wherein the hollow member is arranged with a sealing part for obtaining a sealed state for the puncture needle in the internal space.

The hollow member includes an opening for allowing movement of the puncture needle when the puncture needle is moved in a puncturing direction. In this case, the seal part is preferably configured by a seal member securely attached so as to cover the opening.

The seal member is formed, for example, into a film-shape, and is securely attached to an end face of the hollow member. The seal member may have at least one part retreated in an opposite direction to the puncturing direction from an end face of the hollow member. In this case, a flange part having an opening is arranged at a position retreated from the end face of the hollow member with respect to the hollow member, and the seal member is securely attached to the flange part, or the seal member is formed into a dome-shape, and the seal member is securely attached to the hollow member so that an apex is at a position retreated from the end face of the hollow member.

The seal member may be porous, or may be made of same or same type of resin material as the hollow member. The same type of resin material refers to material having similar physical property (e.g., melting point) as resin, and having high compatibility with respect to each other since the majority of monomer unit in the polymer match or the monomer unit is similar.

The puncture body has a configuration of being held in the hollow member while closely attaching to an inner surface of the hollow member at one part. In this case, the puncture needle preferably has a portion exposed from the puncture body sealed between a closely attached portion of the puncture body and the hollow member, and the seal member.

The hollow member may further include an additional opening which opens in an opposite direction to the puncturing direction. In this case, the sealing part further includes an additional seal member securely attached so as to cover the additional opening. The hollow member may be arranged with an elastic part for exerting a force in the opposite direction to the puncturing direction with respect to the puncture body when the puncture body is moved in the puncturing direction, and for preventing the puncture body from separating from the opening.

The sealing part does not need to be formed as a separate body from the hollow member, and may be integrally molded to the hollow member.
In this case, the sealing part can be incorporated into the hollow member by one resin molding when forming the hollow member by resin molding.

The lancet of the present invention preferably further includes a separation preventing means for preventing the puncture body from separating from the hollow member after use.

The separation preventing means includes one or more elastic part arranged in the puncture body. The elastic part can select a state in which it is engaged to and a state in which it is not engaged to an inner surface of the hollow member.

The lancet of the present invnetion may further include an analyzing tool. The analyzing tool includes a capillary for holding body fluid such as blood and intercellular lymph. In this case, the puncture needle may be able to pass through the capillary.

The hollow member may be configured including a concave part, communicating to the internal space, for fixing the analyzing tool.

The analyzing tool, for example, includes a working electrode and a counter electrode. In this case, the hollow member is preferably arranged with a pair of leads having one end contacting the working electrode or the counter electrode, and the other end being exposed from the hollow member. The one end is formed into a plate spring-form, and presses the working electrode or the counter electrode. One part of the working electrode and the counter electrode may be exposed.

### BRIEF DESCRIPTION OF THE DRAWINGS

Fig. 1 is a cross-sectional view showing a state in which a lancet according to a first embodiment of the present invention is attached to a puncture device.
Fig. 2 is an exploded perspective view of the lancet according to the first embodiment of the present invention.
Fig. 3 is a cross-sectional view in an assembled state of the lancet shown in Fig. 2.
Fig. 4 is a cross-sectional view showing the main parts for describing the operation of attaching the lancet to the puncture device.
Fig. 5 is a cross-sectional view showing a state in which the lancet is stuck into the skin by the puncture device.
Fig. 6 is a cross-sectional view showing the main parts for describing an operation of detaching the lancet from the puncture device.
Fig. 7 is a cross-sectional view for describing another example of the lancet according to the present invention.
Fig. 8 is a cross-sectional view for describing another further example of the lancet according to the present invention.
Fig. 9 is a cross-sectional view for describing another further example of the lancet according to the present invention.
Fig. 10 is a cross-sectional view for describing another further example of the lancet according to the present invention.
Fig. 11 is a cross-sectional view for describing another further example of the lancet according to the present invention.
Fig. 12 is a partially exploded perspective view showing a lancet according to a second embodiment of the present invention.
Fig. 13 is a cross-sectional view taken along line XIII-XIII of Fig. 12.
Fig. 14 is a cross-sectional view taken along line XIV-XIV of Fig. 12.
Fig. 15 is an entire perspective view showing another example of a biosensor in the lancet according to the present invention.
Fig. 16 is a cross-sectional view taken along line XVI-XVI of Fig. 15.
Fig. 17 is an entire perspective view showing another further example of the lancet according to the present invention.
Fig. 18 is a cross-sectional view taken along line XVIII-XVIII of Fig. 17
Fig. 19 is a cross-sectional view describing a conventional lancet and a method of using the same.
Fig. 20 is a cross-sectional view describing a conventional lancet and a method of using the same.

### DESCRIPTION OF SYMBOLS

- 1, 1', 6A, 6B, 6C, 6D, 7: lancet
- 3, 3', 61A, 61B, 60C, 60D, 70: tubular member (hollow member)
- 30, 67C, 71: internal space
- 32, 63A, 63B, 61C, 77: opening
- 36, 62A, 62B: end face
- 4, 4': puncture body
- 41, 41': puncture needle
- 45': elastic part (separation preventing means)
- 5, 60A, 60B, 63C, 73B: seal member (sealing part)
- 5': sealing part
- 62C, 72: opening (additional opening)
- 64C, 73A: seal member (additional seal member)
- 74: concave part (of tubular member)
- 75: lead
- 78: end (other one of the lead)
- 79: end (one of the lead)
- 8: biosensor (analyzing tool)
- 83: capillary (of biosensor)
- 84: working electrode (of biosensor)
- 85: counter electrode (of biosensor)
- N1: puncturing direction

### BEST MODE FOR CARRYING OUT THE INVENTION

The lancet according to the present invention will be described below as first and second embodiments with reference to the drawings.

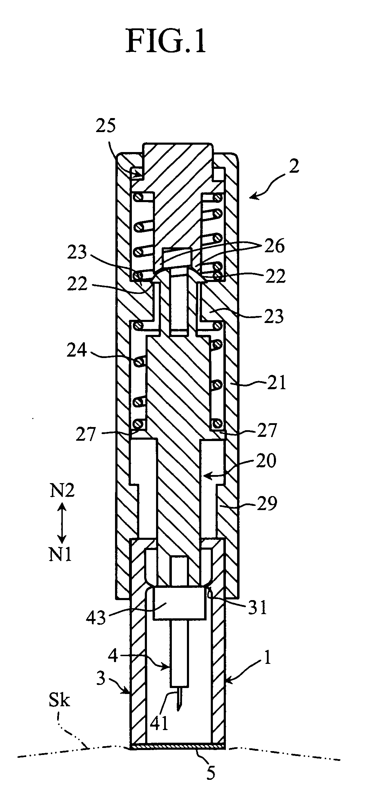

First, the first embodiment of the present invention will be described with reference to Fig. 1 to Fig. 6.

As shown in Fig. 1, a lancet 1 according to the present invention is used by being attached to a puncture device 2 including a lancet holder 20 for holding the lancet 1.

The lancet holder 20 is accommodated inside a housing 21 to be movable in N1, N2 directions, and is configured to be biased towards the N1 direction by a coil spring 24 by engaging a pair of latch parts 22 to a convex part 23 of the housing 21.

The engagement state of the latch part 22 is released by a pressing operation of an operation cap 25. That is, the operation cap 25 is slidable in the N1, N2 directions with respect to the housing 21, where a pair of projections 26 interfere with the pair of latch parts 22 when the operation cap 25 is moved in the N1 direction. Each of the pair of latch parts 22 is thereby displaced inward, and the engagement state of the latch part 22 is released.

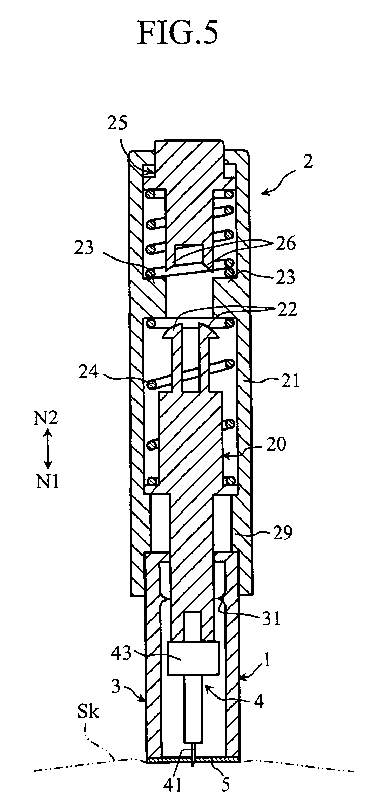

The coil spring 24 is arranged between the convex part 23 of the housing 21 and a convex part 27 of the lancet holder 20, and is compressed when the latch part 22 of the lancet holder 20 is engaged to the convex part 23 of the housing 21. Thus, the lancet holder 20 is biased in the N1 direction by the coil spring 24 when the latch part 22 is engaged with the convex part 23. When the engagement of the latch part 22 to the convex part 23 is released, the lancet holder 20 is moved in the N1 direction by the elastic force of the coil spring 24, and a puncture needle 41 of the lancet 1 pierces the skin Sk (see Fig. 5).

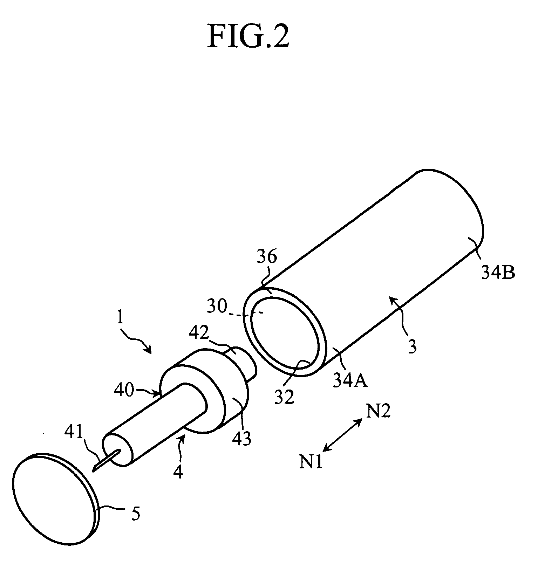

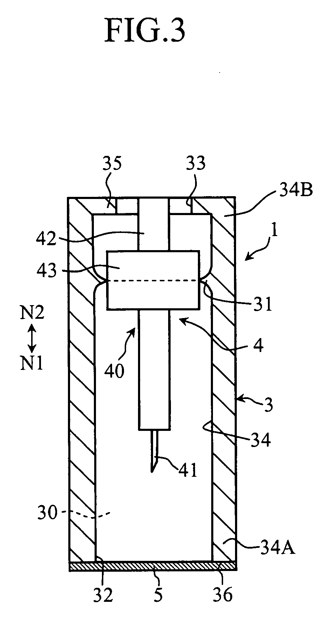

As shown in Fig. 2 and Fig. 3, the lancet 1 includes a tubular member 3, a puncture body 4, and a seal member 5.

The tubular member 3 accommodates and holds the puncture body 4 in an internal space 30. The tubular member 3 has an engagement part 31 and openings 32, 33, and is integrally formed entirely through resin forming and the like. The resin for forming the tubular member 3 may be polyethylene, polypropylene, polycarbonate, polystyrene, polyoxymethylene, or polyacryl.

The engagement part 31 holds the puncture body 4 in the internal space 30 of the tubular member 3 by engaging a bulging part 43 of the puncture body 4 when not in use, and annularly projects towards the inner side. The engagement part 31 also restricts the puncture body 4 along with a flange part 35, to be hereinafter described, after use. The opening 32 allows the movement of the puncture body 4 (projection of puncture needle 41) when the puncture body 4 is moved in the puncturing direction N1, and is opened at an end 34A on the puncturing direction N1 side of the tubular member 3. The opening 33 allows the movement of the lancet holder 20 (see Fig. 1) in the puncture device 2. The opening 33 is defined by the flange part 35, and is opened at an end 34B on a retreating direction N2 side of the tubular member 3.

The puncture body 4 is moved in the N1, N2 directions by the lancet holder 20 (see Fig. 1) of the puncture device 2, and includes a main body 40 and the puncture needle 41.

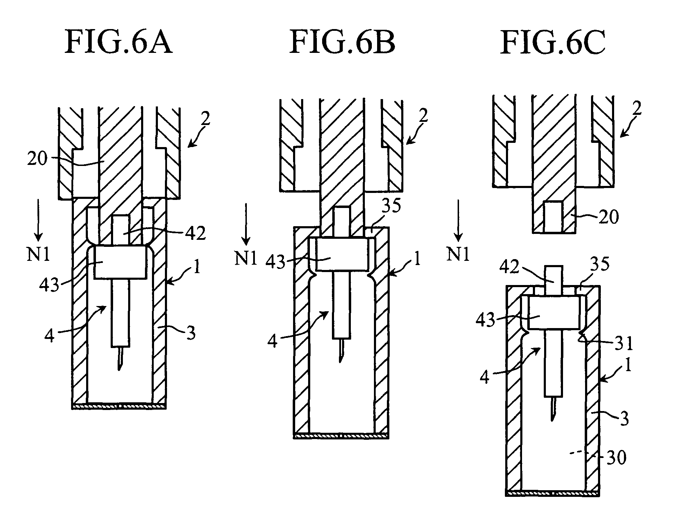

The main body 40 holds the puncture needle 41 and includes a fit-in part 42 and the bulging part 43. The fit-in part 42 is a portion to be held by the lancet holder 20 of the puncture device 2. The bulging part 43 is a portion to be engaged with the engagement part 31 of the tubular member 3 when not in use, where the puncture body 4 is accommodated in the internal space 30 of the tubular member 3 by engaging the bulging part 43 to the engagement part 31. The engagement force of the bulging part 43 and the engagement part 31 is smaller than the fit-in force when the fit-in part 42 is fitted into the lancet holder 20. The magnitude relation of the engagement force and the fit-in force is realized by, for example, ensuring a large friction force between the fit-in part 42 and the lancet holder 20, or by arranging a convex part on either the fit-in part 42 or the lancet holder 20 and arranging a concave part on the other one to engage with the convex part and ensuring a large engagement force between the convex part and the concave part.
The bulging part 43 engages the engagement part 31 at the entire periphery since the engagement part 31 is annularly formed. Thus, when the lancet 1 is not in use, the bulging part 43 closely attaches to the engagement part 31 without a gap. The bulging part 43 is also a portion restrained between the engagement part 31 and the flange part 35 after use. The puncture body 4 is prevented from slipping out from the openings 32, 33 by restraining the bulging part 43 between the engagement part 31 and the flange part 35 (see Fig. 6C). Such main body 40 is integrally formed entirely by resin molding.

The puncture needle 41 pierces the skin Sk and incises the skin Sk (see Fig. 5), and is integrated with respect to the main body 40 through insert molding and the like. Such puncture needle 41 is formed by grinding a metal wire rod and forming a blade surface.

The seal member 5 accommodates the puncture needle 41 in a sealed state in the internal space 30 of the tubular member 3, and is securely attached to an end face 36 of the tubular member 3 so as to seal the opening 32 of the tubular member 3. When the seal member 5 is securely attached to the end face 36 of the tubular member 3, the puncture needle 41 is held in the sealed space since the engagement part 31 of the tubular member 3 and the bulging part 43 of the puncture body 4 are closely attached without a gap. Thus, when sterilizing the puncture needle 41 by γ ray, X ray, electron ray irradiation, or the like after manufacturing the lancet 1, such sterilized state can be appropriately maintained.

Metal thin film such as aluminum foil may be used for the seal member 5, but a member made of resin is preferably used, which thickness is between 1 and 200µm, or preferably between 2 and 10µm. The seal member 5 will not be carelessly broken when operating the lancet 1 in an unused state and will be reliably broken by the puncture needle 41 when puncturing by setting the thickness of the seal member 5 to the above range.

The resin material for forming the seal member 5 may be polyethylene, polyester (e.g., polyethylene terephtalate), nylon, polystyrene, polyvinyl chloride, polyvinylidene chloride, and ethylene-acetate copolymer. The seal member 5 may be formed only from the mentioned resin materials, but may be formed by joining a film made from a different resin material and vapor depositing metal material such as aluminum on the surface of the film formed by the above resin or laminating a metal film made of aluminum etc. on the resin film.

The seal member 5 may be made from the same or same type of resin material as the tubular member 3 such as polyethylene or polypropylene. Thus, when securely attaching the seal member 5 to the end face 36 of the tubular member 3, the material may be selected in view of the joining property between the seal member 5 and the tubular member 3, or the secure attachment conditions do not need to be closely examined and the seal member 5 can be easily and reliably securely attached to the tubular member 3 by simply thermal pressing.

The porous seal member 5 may be used. Porous in this case refers to porous of an extent that the sterilized state can be maintained after sterilizing the puncture body 4, and also refers to porous having a bore diameter (e.g., smaller than or equal to 0.45µmm) of an extent that the fungus body does not pass through and gas such as air passes through. When such seal member 5 is used, gas sterilization (e.g., ethylene oxide gas sterilization) is adopted for a method of sterilizing the puncture needle 41 after manufacturing the lancet. In other words, the puncture needle 41 can be sterilized using a simple and inexpensive device without using a complex and expensive device capable of irradiating γ ray, X ray, or electron ray.

When securely attaching the seal member 5 to the end face 36 of the tubular member 3, secure attachment is carried out normally by arranging a rib on the end face 36 of the tubular member 3, and melting the rib by ultrasonic wave, heat, or the like. Thus, if porous seal member 5 is used, the components enter the hole of the seal member 5 when the rib is melted, whereby the seal member 5 is reliably and strongly attached to the end face 36 of the tubular member 3.

The material for forming the porous seal member 5 may be polycarbonate, polysulfone, polyethersulfone, polyethylene, cellulose acetate, nitrocellulose, polyvinylidene difluoride, polypropylene, polytetrafluoroethylene, nylon, and polyester.

The method of using the lancet 1 and the method of incising the skin will now be described.

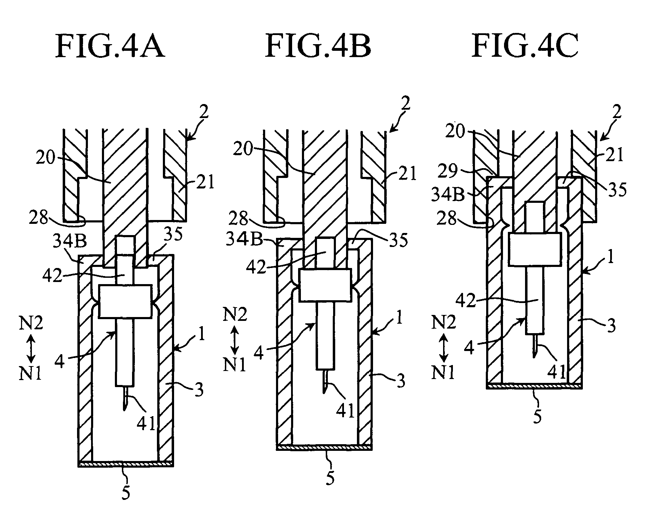

When incising the skin Sk, the lancet 1 is first attached to the puncture device 2, as shown in Fig. 4A to Fig. 4C. The attachment of the lancet 1 is carried out by pushing the end 34B formed with the flange part 35 in the lancet 1 into the end opening 28 of the housing 21 in the puncture device 2. In this case, the end 34B of the tubular member 3 of the lancet 1 is fitted into the housing 21 of the puncture device 2, and the puncture body 4 of the lancet 1 is fitted into the lancet holder 20 in the fit-in part 42. The lancet 1 has the puncture needle 41 sealed by the seal member 5, and thus the puncture needle 41 is not exposed when the lancet 1 is attached to the puncture device 2, whereby the user does not feel a sense of fear.

The lancet holder 20 is moved in the N2 direction when pushing the lancet 1 into the housing 21. Thus, as shown in Fig. 1, the latch part 22 of the lancet holder 20 is engaged with the convex part 23 of the housing 21, and the elastic force is accumulated in the coil spring 24. The movement of the lancet holder 20 and the puncture body 4 is limited when the latch part 22 is engaged with the convex part 23. Thus, as shown in Fig. 4B and Fig. 4C, the tubular member 3 is moved in the N2 direction independent from the lancet holder 20 and the puncture body 4. The movement of the tubular member 3 is limited when the tubular member 3 interferes with a stopper 29 of the housing 21. The engagement state of the tubular member 3 and the puncture body 4 is thereby released in the lancet 1 since the tubular member 3 moves in the N2 direction independent from the puncture body 4.

Obviously, the lancet 1 may be attached with the latch part 22 of the lancet holder 20 engaged in advance with the convex part 23 of the housing 21.

The puncture body 4 is then moved in the N1 direction, and the puncture needle 4 is stuck into the skin Sk, as shown in Fig. 1 and Fig. 5. This operation is carried out by having the user move the operation cap 25 of the puncture device 2 in the N1 direction with respect to the housing 21. When the operation cap 25 is moved in the N1 direction, the projection 26 of the operation cap 25 interferes with the latch part 22, and the state in which the latch part 22 is engaged with the convex part 23 is released. The force then acts in the N1 direction with respect to the lancet holder 20 by the elastic force of the coil spring 24. The puncture body 4 is thereby relatively moved in the N1 direction with respect to the tubular member 3. The movement in the N1 direction of the lancet holder 20 and the puncture body 4 is performed until the lancet holder 20 reaches a position interfering with the stopper 29. The puncture needle 41 consequently breaks the seal member 5 and projects out from the tubular member 3, and the puncture needle 41 of the puncture body 4 pierces the skin Sk thereby incising the skin Sk.

The user does not need to actively strip the seal member 5 in time of puncturing by breaking the seal member 5 with the puncture needle 41, and thus the load on the user can be alleviated in such regards.

After the incision of the skin Sk is terminated, the lancet 1 is detached from the puncture device 2, as shown in Figs. 6A to 6C. The lancet 1 can be detached by exerting a force in the puncturing direction N1 on the lancet 1. When the lancet 1 is relatively moved in the N1 direction with respect to the puncture device 2, the tubular member 3 relatively moves in the N1 direction with respect to the puncture device 2, as shown in Fig. 6A. In this case, the fit-in part 42 of the puncture body 4 is held by the lancet holder 20, and the fit-in force between the fit-in part 42 and the lancet holder 20 is made larger than the engagement force between the bulging part 43 and the tubular member 3, whereby the tubular member 3 relatively moves in the N1 direction with respect to the puncture body 4. When the tubular member 3 is moved by a constant distance, the bulging part 43 of the puncture body 4 interferes with the flange part 35 of the tubular member 3 as shown in Fig. 6B. Accordingly, the puncture body 4 moves in the N1 direction along with the tubular member 3, and the fit-in part 42 of the puncture body 4 is removed from the lancet holder 20. As a result, the bulging part 43 of the puncture body 4 is restrained between the flange part 35 and the engagement part 31 in the internal space 30 of the tubular member 3. Thus, when the lancet 1 is detached from the puncture device 2 after using the lancet 1, the puncture body 4 will not slip out from the tubular member 3 and thus is safe.

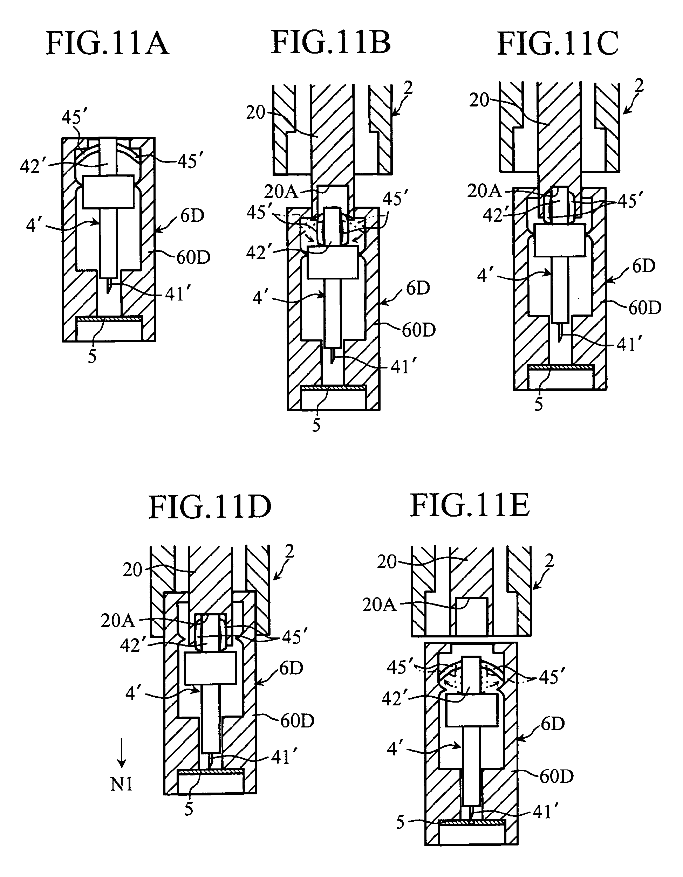

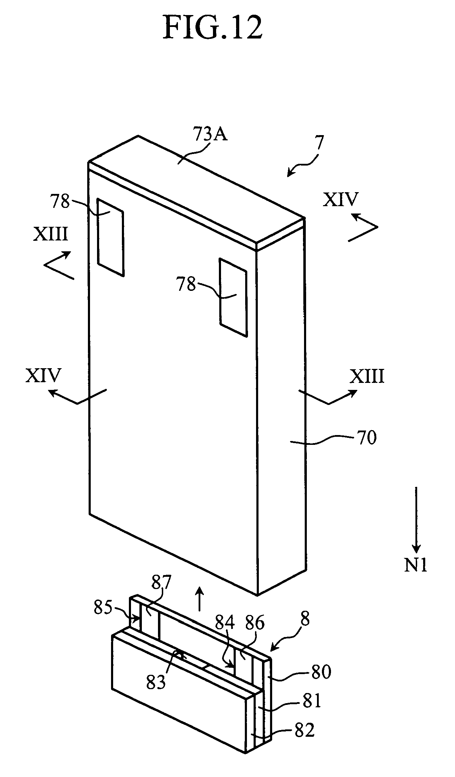

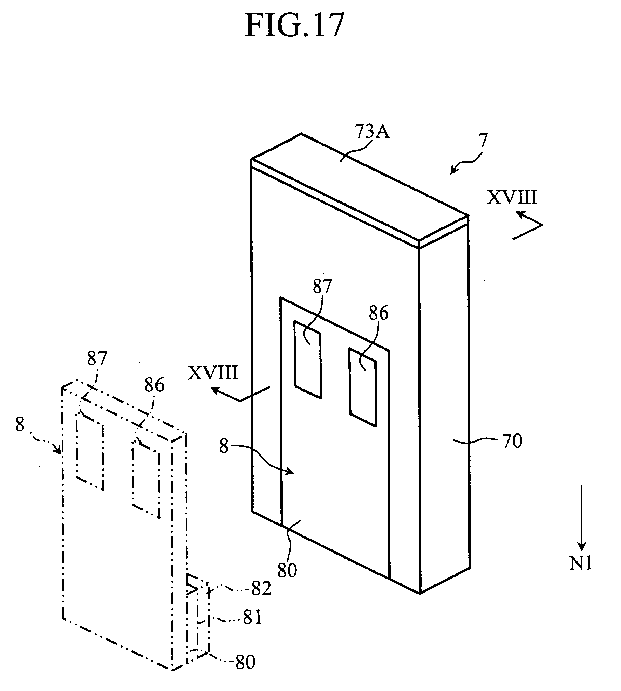

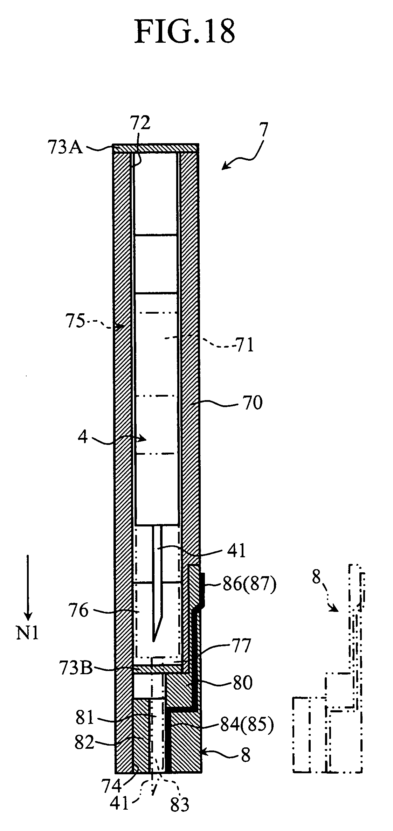

In the lancet 1, there is no need to detach the cover 82 of the puncture device 8 and attach the lancet 9 to the lancet holder 80 before puncturing, and to detach the cover 82 and detach the lancet 9 from the lancet holder 80 after puncturing as in the prior art described with reference to Fig. 17 and Fig. 18. The blood drawing using the lancet 1 shown in Fig. 1 to Fig. 6 is carried out with a simple operation, and the load of the user is alleviated. In the lancet 1 shown in Fig. 1 to Fig. 6, the operation of detaching the cap 92 from the lancet 9 after attaching the lancet 9 to the puncture device 8 as in the prior art is not necessary (see Fig. 11 and Fig. 12), and thus the load of the user is alleviated in such regards.

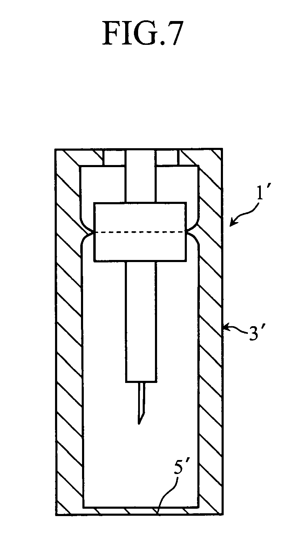

Various modifications may be made on the present invention, and for example, the sealing part 5' corresponding to the seal member 5 (see Fig. 2) may be integrally formed with a tubular member 3' as in a lancet 1' shown in Fig. 7 without forming the seal member 5 and the tubular member 3 as separate bodies. The thickness of the sealing part 5' in this case is about the same as the seal member (see Fig. 2). In such lancet 1', the sealing part 5' can be incorporated into the tubular member 3' in one resin molding when forming the tubular member 3' by resin molding, and thus the seal member 5 does not need to be formed separate from the tubular member 3 and the seal member 5 does not need to be securely attached to the tubular member 3 as in the lancet 1 shown in Fig. 3, which is advantageous in manufacturing.

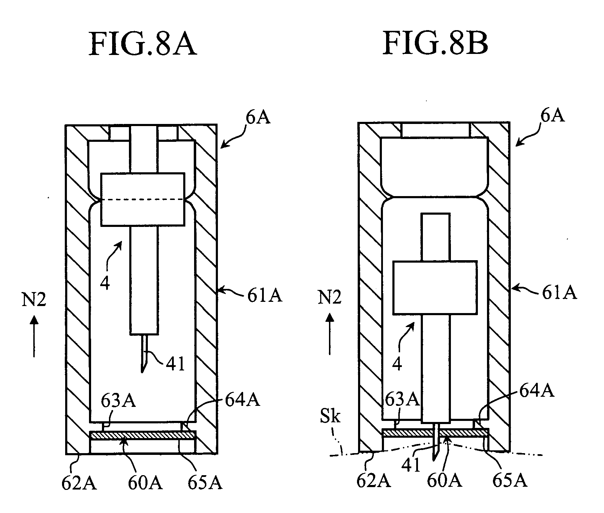

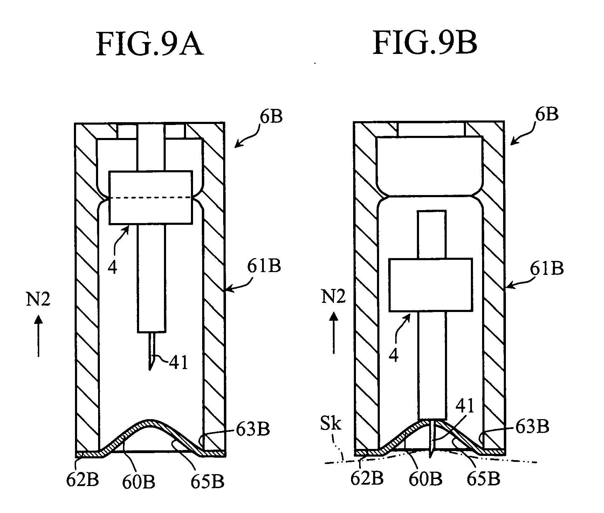

A configuration in which seal members 60A, 60B are retreated from end faces 62A, 62B of tubular members 61A, 61B as in lancets 6A, 6B shown in Fig. 8 and Fig. 9 may be adopted. More specifically, the lancet 6A shown in Fig. 8A and Fig. 8B is arranged with a flange part 64A having an opening 63A at a position retreated in the N2 direction from the end face 62A of the tubular member 61A, where the seal member 60A is securely attached to the flange part 64A. The lancet 6B shown in Fig. 9A and Fig. 9B forms the seal member 60B to a dome-shape to seal an opening 63B with the apex of the dome retreated in the N2 direction from the end face 62B of the tubular member 61B.

In the lancets 6A, 6B shown in Fig. 8 and Fig. 9, the portion to be broken by the puncture needle 41 is retreated in the N2 direction from the end faces 62A, 62B of the tubular members 61A, 61B, and thus when the skin Sk is incised and the blood comes out, the blood is prevented from spreading along surfaces 65A, 65B of the seal members 60A, 60B. Thus, the appropriate sphere-shaped blood comes out from the skin Sk by surface tension, whereby blood can be easily and reliability drawn.

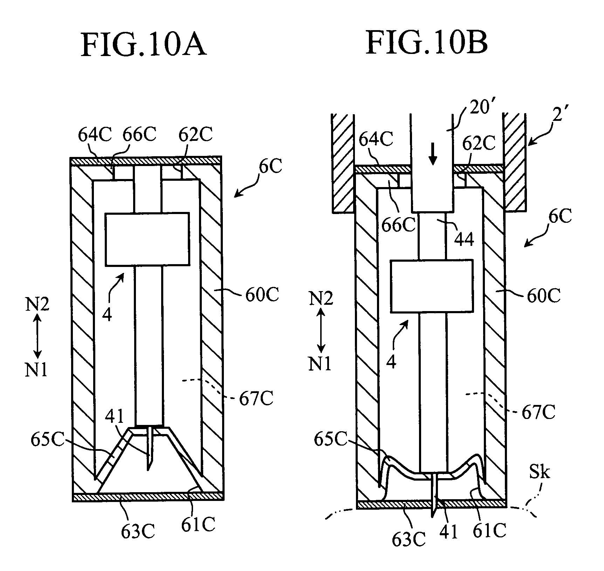

As shown in Fig. 10, a configuration in which both openings 61C, 62C of a tubular member 60C are sealed by seal members 63C, 64C is adopted for the lancet 6C. In the lancet 6C, an elastic part 65C and a flange part 66C are arranged so that the puncture body 4 does not slip out from the tubular member 60C and the puncture needle 41 does not project out in non-use and after use. The elastic part 65C is configured by a plate spring, a coil spring, and the like.

While such lancet 6C is attached to a puncture device 2', the puncture body 4 does not couple with the components of the puncture device 2', for instance, the seal member 64C is broken by a hammer 20' of the puncture device 2', and a load in the N1 direction is input to an end 44 of the puncture body 4. The puncture body 4 of the lancet 6C is then moved towards the skin Sk. When the load from the hammer 20' on the puncture body 4 is released, the puncture body 4 is moved in the retreating direction N2 by the elastic force of an elastic part 65C. The puncture needle 41 of the puncture body 4 is accommodated in an internal space 67C of the tubular member 60C, and the puncture body 4 is held between the elastic part 65C and the flange part 66C. As a result, the puncture body 4 is prevented from slipping out from the tubular member 60C after use in the lancet 6C.

The hammer 20' of the puncture device 2' is preferably formed into a shape the distal end thereof can easily break the seal member 64, such as tapered shape. A configuration of being broken by the hammer 20' does not necessarily need to be adopted for the seal member 64C, and the seal member 64C may be made from an elastic sheet, and the load may be input to the puncture body 4 of the lancet 6C from the hammer 20' by elastic deformation.

As shown in Fig. 11A, a plurality of elastic parts 45' may be arranged in a fit-in part 42' of a puncture body 4'. The elastic part 45' engages the inner surface of a tubular member 60D when outer force is not acting. As shown in Fig. 11B and Fig. 11C, the plurality of elastic parts 45' are displaced inward when the fit-in part 42' of the puncture body 4' is fitted into the lancet holder 20 in the puncture device 2, and accommodated in a concave part 20A of the lancet holder 20. The plurality of elastic parts 45' are biased in an outward spreading direction.

In such lancet 6D, the puncturing operation is performed with the plurality of elastic parts 45' biased by the lancet holder 20, as shown in Fig. 11D. After the puncturing operation is terminated, the lancet 4' is detached from the lancet holder 20, as shown in Fig. 11E, in which case the plurality of elastic parts 45'are elastically recovered. The plurality of elastic parts 45' are then again engaged with the inner surface of the tubular member 60D, and the puncture body 4' is held inside the tubular member 60D. Thus, in the lancet 6D, the puncture body 4' is prevented from slipping out from the tubular member 60D or the puncture needle 41' from being exposed from the tubular member 60D after puncturing.

A configuration in which the opening at the end on the opposite direction of the puncturing direction is sealed with a sealing part such as a seal member may be adopted in the lancets 1, 1', 6A, 6B, and 6D shown in Figs. 3, 7, 8, 9, and 11. The sealing part in this case merely needs to be broken by the lancet holder when attaching the lancet to the puncture device, for example, or a weak part with small thickness compared to other portions may be provided to the sealing part, and a sharp portion may be arranged at the end of the lancet holder so that the sealing part can be easily broken by the lancet holder.

In the present invention, the means for preventing the lancet from slipping out from the tubular member after the lancet is used is not limited to those described above, and other configurations may be adopted.

A mode of puncturing the skin Sk by breaking the seal member 5, 60A, 60B, 63C with the puncture needle 41, 41' does not necessarily need to be adopted in the lancet of the present invnetion, and the seal member 5, 60A, 60B, 63C may be stripped for use.

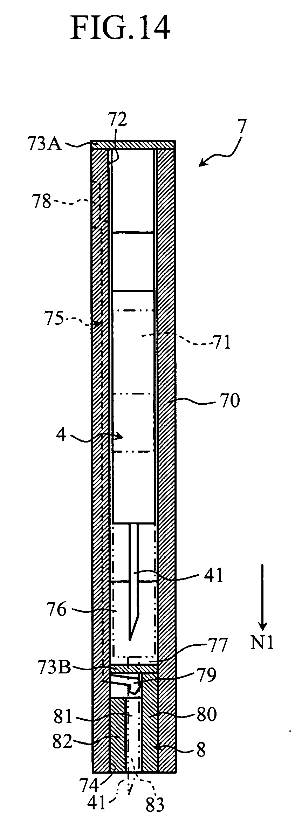

A lancet according to the second embodiment of the present invention will now be described with reference to Figs. 12 to 14. In these figures, same reference numerals are denoted for same elements as in the lancet 1 previously described with reference to Figs. 1 to 6, and redundant description will be omitted below.

A lancet 7 shown in Figs. 12 to 14 accommodates the puncture body 4 in an internal space 71 of a tubular member 70 and seals an upper opening 72 of the tubular member 70 with a seal member 73A. The tubular member 70 is formed into a square tubular shape by resin and the like. The tubular member 70 is formed with a concave part 74, and is fixed with a pair of leads 75.

The concave part 74 is a portion to be attached with the biosensor 8, and allows movement of the puncture needle 41 of the puncture body 4.
The concave part 74 is communicated to the internal space 71 by way of a communication hole 76. A lower opening 77 of the communication hole 76 is sealed by a seal member 73B.

The biosensor 8 electrochemically measures specific component (e.g., glucose, cholesterol, or lactic acid) in the blood, and has a configuration in which a spacer 81 is attached to a substrate 80 by way of a cover 82. The biosensor 8 is arranged with a capillary 83 for aspirating and holding blood. The capillary 83 is a portion through which the puncture needle 41 of the puncture body 4 can be moved. The capillary 83 is arranged with a reagent layer (not shown). The substrate 80 is arranged with a working electrode 84 and a counter electrode 85 for applying voltage and measuring current value. Ends 86, 87 of the working electrode 84 and the counter electrode 85 are exposed from the biosensor 8.

The pair of leads 75 are provided to achieve conduction between a connector (not shown) arranged in the puncture device and the working electrode 84 and the counter electrode 85 in the biosensor 8. Each lead 75 is embedded in the tubular member 70 by insert molding and the like. End 78 of the lead 75 is exposed from the surface of the tubular member 60. Thus, the connector (not shown) arranged in the puncture device and the lead 75 can be brought into contact with each other by way of the end 78. End 79 is formed into a plate spring form, and is brought in contact with the ends 86, 87 of the working electrode 84 and the counter electrode 85 of the biosensor 8.

Such lancet 7 is used by being attached to the puncture device. The seal member 73A of the lancet 7 may be stripped by the user before being attached to the puncture device, or may be broken during puncturing by an element (e.g., lancet holder or hammer) of the puncture device.

When the lancet 7 is attached to the puncture device, the connector of the puncture device contacts the end 78 of the lead 75 in the lancet 7.
The voltage thus can be applied between the working electrode 84 and the counter electrode 85 of the biosensor 8 via the connector and the lead 75, and a response current in time of voltage application can be measured.

After the lancet 7 is attached to the puncture device, the puncture body 4 is moved in the N1 direction by the element (e.g., lancet holder or hammer) of the puncture device. The puncture needle 41 then passes through the capillary 83 of the biosensor 8 and punctures the skin, whereby blood flows out from the skin. After puncturing, the puncture needle 41 is removed from the skin. As the puncture needle 41 passes through the capillary 83 and punctures the skin, the bleeding site from the skin is the position corresponding to the capillary 83. Thus, the blood flowed out from the skin is appropriately introduced into the capillary 83 of the biosensor 8. In the puncture device, voltage is applied between the working electrode 84 and the counter electrode 85, the response current at this point is measured, and the concentration of a specific component in the blood is measured based on the response current.

Such lancet 7 can be formed by separately forming the biosensor 8 and other portions, and then fixing the biosensor 8 to the concave part 74 of the tubular member 70. The fixation of the biosensor 8 to the concave part 74 may use the fit-in force in the concave part 74 and the spring elasticity at the end 79 of the lead 75, or may use adhesive and the like.

In the lancet 7, the upper opening 72 of the tubular member 70 is sealed with the seal member 73A, and the lower opening 77 of the communication hole 76 is sealed with the seal member 73B, whereby air tightness of the internal space 71 is ensured. The puncture body 4 is accommodated in the internal space 71. Thus, if sterilization is performed with the puncture body 4 accommodated in the internal space 71 in which air tightness is ensured, the sterilized state of the puncture body 4 (puncture needle 41) can be maintained. Furthermore, the biosensor 8 can be fixed to the concave part 74 of the tubular member 70 after sterilizing the puncture needle 41, and thus the puncture needle 41 will not be contaminated by incorporating the biosensor 8 into the lancet 7.

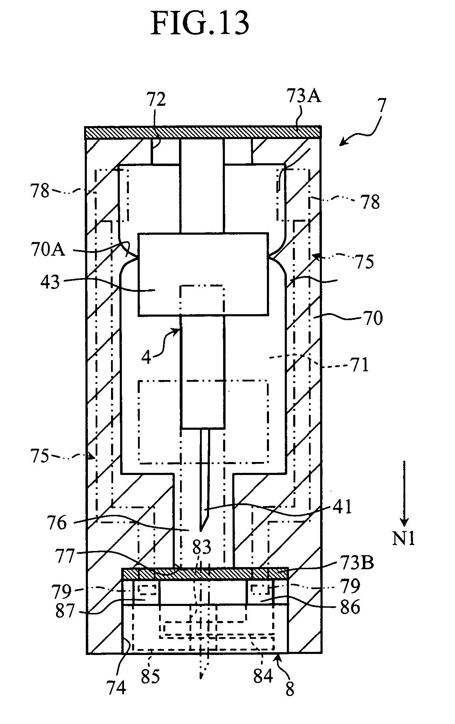

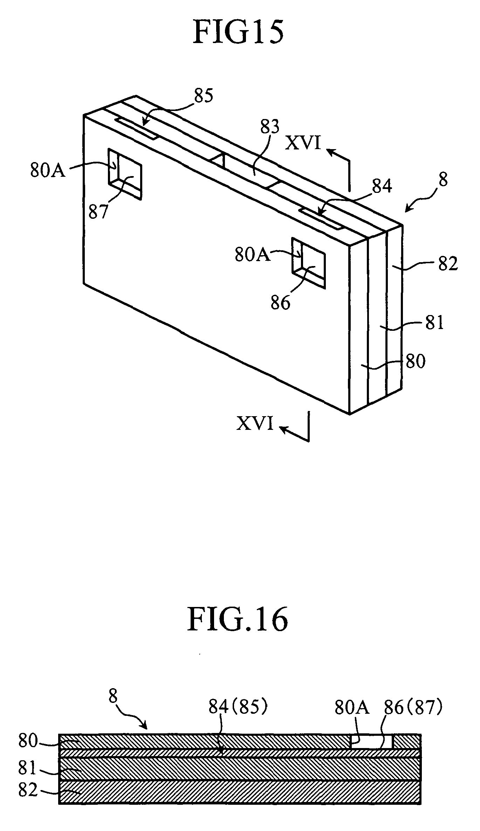

The lancet 7 including the biosensor 8 is not limited to the mode described above, and various changes can be made. For instance, the tubular member 70 may be formed into a cylindrical shape, the seal member 73A may be omitted, the bulging part 43 of the puncture body 4 may be closely attached to an engagement part 70A of the tubular member 70, and the sterilized state of the puncture needle 41 may be maintained by a sealed space between the closely attached portion of the bulging part 43 and the engagement part 70A and the seal member 73B (see Fig. 13). The biosensor 8 is not limited to a mode in which the ends of the working electrode 84 and the counter electrode 85 are exposed at the side of the cover 82, and the working electrode 84 and the counter electrode 85 may be formed on a surface defining the capillary 83 in the substrate 80 and the ends 86, 87 of the working electrode 84 and the counter electrode 85 may be exposed by forming a pass-through hole 80A in the substrate 80, as shown in Fig. 15 and Fig. 16.

As shown in Fig. 17 and Fig. 18, the lead fixed to the tubular member 70 may be omitted, and the ends 86, 87 of the working electrode 84 and the counter electrode 85 of the biosensor 8 may be exposed from the back surface of the substrate 80, so that the connector of the puncture device contacts the ends 86, 87.

## Claims

1. A lancet comprising a puncture body with a puncture needle, and a hollow member having an internal space for accommodating the puncture body, wherein
the hollow member is arranged with a sealing part for obtaining a sealed state for the puncture needle in the internal space.

2. The lancet according to claim 1, wherein
the hollow member includes an opening for allowing movement of the puncture needle when the puncture needle is moved in a puncturing direction; and
the seal part includes a seal member securely attached so as to cover the opening.

3. The lancet according to claim 2, wherein the seal member is formed into a film-shape, and is securely attached to an end face of the hollow member.

4. The lancet according to claim 2, wherein the seal member has at least one part retreated in an opposite direction to the puncturing direction from an end face of the hollow member.

5. The lancet according to claim 4, wherein
the opening is defined by a flange part arranged at a position retreated in the opposite direction to the puncturing direction from the end face of the hollow member; and
the seal member is formed into a film-shape, and is securely attached to the flange part.

6. The lancet according to claim 4, wherein the seal member is formed into a dome-shape, and is securely attached to the hollow member so that an apex is at a position retreated in the opposite direction to the puncturing direction from the end face of the hollow member.

7. The lancet according to claim 2, wherein the seal member is porous.

8. The lancet according to claim 2, wherein the hollow member and the seal member are made of same or same type of resin material.

9. The lancet according to claim 2, wherein
the puncture body is held in the hollow member while closely attaching to an inner surface of the hollow member at one part; and
the puncture needle has a portion exposed from the puncture body sealed between a closely attached portion of the puncture body and the hollow member, and the seal member.

10. The lancet according to claim 2, wherein
the hollow member has an additional opening which opens in an opposite direction to the puncturing direction; and
the sealing part further includes an additional seal member securely attached so as to cover the additional opening.

11. The lancet according to claim 10, wherein the hollow member is arranged with an elastic part for exerting a force in the opposite direction to the puncturing direction with respect to the puncture body when the puncture body is moved in the puncturing direction, and for preventing the puncture body from separating from the opening.

12. The lancet according to claim 1, wherein the sealing part is integrally molded to the hollow member.

13. The lancet according to claim 1, further comprising a separation preventing means for preventing the puncture body from separating from the hollow member after use.

14. The lancet according to claim 13, wherein the separation preventing means includes one or more elastic part arranged in the puncture body.

15. The lancet according to claim 14, wherein the one or more elastic part selects a state in which the elastic part is engaged to and a state in which the elastic part is not engaged to an inner surface of the hollow member.

16. The lancet according to claim 1, further comprising an analyzing tool.

17. The lancet according to claim 16, wherein
the analyzing tool includes a capillary for holding body fluid; and
the puncture needle can pass through the capillary.

18. The lancet according to claim 16, wherein the hollow member includes a concave part, communicating to the internal space, for fixing the analyzing tool.

19. The lancet according to claim 16, wherein
the analyzing tool includes a working electrode and a counter electrode; and
the hollow member is arranged with a pair of leads having one end contacting the working electrode or the counter electrode, and the other end being exposed from the hollow member.

20. The lancet according to claim 19, wherein the one end is formed into a plate spring-form, and presses the working electrode or the counter electrode.

21. The lancet according to claim 16, wherein
the analyzing tool includes a working electrode and a counter electrode; and
one part of the working electrode and the counter electrode is exposed.
